Europäisches Patentamt

(19) European Patent Office

Office européen des brevets

(11) EP 0 550 343 B1

# (12) FASCICULE DE BREVET EUROPEEN

(45) Date de publication et mention
de la délivrance du brevet:
**10.01.1996 Bulletin 1996/02**

(51) Int. Cl.6: **A61N 1/36**, A61N 1/38,
A61B 5/046

(21) Numéro de dépôt: **92403579.3**

(22) Date de dépôt: **30.12.1992**

(54) **Procédé et système d'analyse de l'activité cardiaque pour dispositif implantable de traitement des tachycardies**

Verfahren und System zur Analyse der Herztätigkeit für implantierbare Vorrichtung zur Behandlung von Tachykardien

Process and system for the analysis of the cardiac activity for implantable treatment device of tachycardia

(84) Etats contractants désignés:
**BE CH DE ES FR GB IT LI SE**

(30) Priorité: **31.12.1991 FR 9116365**

(43) Date de publication de la demande:
**07.07.1993 Bulletin 1993/27**

(73) Titulaire: **ELA MEDICAL (Société anonyme)**
**F-92541 Montrouge Cédex (FR)**

(72) Inventeurs:
• **Nitzsche, Rémi**
  **F-78650 Beynes (FR)**
• **Limousin, Marcel**
  **F-92120 Montrouge (FR)**
• **Jacobson, Peter**
  **F-67500 Haguenau (FR)**

(74) Mandataire: **Laget, Jean-Loup**
**F-75116 Paris (FR)**

(56) Documents cités:
  **EP-A- 0 395 242          US-A- 3 946 725**

• **COMPUTERS IN CARDIOLOGY, IEEE, 7 Octobre 1986, BOSTON, US, pages 163 - 166 R.A. DUFAULT ET AL, 'DUAL LEAD FIBRILLATION DETECTION FOR IMPLANTABLE DEFIBRILLATORS VIA LMS ALGORITHM'**
• **MEDIZINTECHNIK, vol. 24, no. 3, Septembre 1984, BERLIN, DE, pages 84 - 91 K.B. OTTE ET AL 'Physiologische Elektrostimulation des Herzens Stand und Entwicklungsaussichten'**

## Description

L'invention concerne les systèmes de traitement des arythmies cardiaques, plus particulièrement ceux destinés à la réduction des tachycardies. Un tel système peut être partie intégrante d'un stimulateur cardiaque à visée antiarythmique, ou d'un défibrillateur implantable.

Les tachycardies peuvent se classer en deux catégories distinctes suivant le foyer d'origine de ces accélérations pathologiques du rythme cardiaque. On distingue les tachycardies supra-ventriculaires (TSV) dont l'origine se situe au niveau de l'oreillette et les tachycardies ventriculaires (TV) dont le foyer se localise dans le ventricule. A chacun de ces types de tachycardie correspond un mode approprié de traitement par émission d'énergie électrique. Pour les TSV plusieurs modes de traitement sont possibles depuis l'absence de traitement spécifique jusqu'à l'émission d'impulsions dont les énergies sont limitées à quelques micro-joules.

Pour les TV il est parfois nécessaire de délivrer un choc électrique de quelques joules le plus tôt possible après l'observation du phénomène.

De tels chocs électriques sont en général pénibles et douloureux pour les patients et doivent être délivrés à bon escient.

Il est donc important de distinguer lors de l'analyse des signaux cardiaques, en cas de détection d'une accélération du rythme cardiaque, si l'on a affaire à une TSV ou à une TV. Cette distinction est difficile pour les appareils qui ne détectent l'occurrence d'une tachycardie qu'à partir de signaux recueillis dans le ventricule.

Le brevet FR 2.598.920 décrit un système où le signal cardiaque est recueilli dans le ventricule et détecte les accélérations et décélérations du rythme cardiaque. Suivant l'analyse de ce rythme et des séquences d'accélération et décélération, l'appareil évalue la présence d'une arythmie susceptible d'être traitée. Le brevet EP 0 360 412 base son interprétation du type de tachycardie sur un classement, en plusieurs régions contiguës, des rythmes analysés à partir du signal cardiaque détecte au niveau du ventricule.

Ces deux systèmes présentent l'inconvénient de se fier uniquement aux signaux recueillis dans une seule cavité cardiaque.

Le brevet US 4.860.749 décrit un appareil recueillant les signaux représentatifs de l'activité cardiaque à la fois dans le ventricule et dans l'oreillette. A l'aide d'un algorithme faisant intervenir plusieurs seuils de fréquence et la comparaison à des délais introduits pour chaque patient dans l'appareil, celui-ci définit différents types de tachycardies.

Toutefois, en raison des nombreux tests qui doivent être effectués, un tel algorithme est difficile à mettre en oeuvre dans un appareil implantable.

Un but de la présente invention est de proposer un système incorporable dans un stimulateur cardiaque ou un défibrillateur implantable capable de recueillir simultanément l'activité cardiaque au niveau de l'oreillette et du ventricule afin d'établir, par des moyens logiciels simples de mise en oeuvre, une distinction entre TSV et TV pour la commande du mode de thérapie approprié.

Un autre but de l'invention est de proposer un procédé d'analyse de l'activité cardiaque pour établir une distinction entre les types de tachycardie, TV ou TSV, dans un délai court.

L'invention a pour objet un procédé d'analyse de l'activité cardiaque, pour dispositif implantable de traitement des tachycardies, du type dans lequel sont analysés les signaux en provenance de l'oreillette et les signaux en provenance du ventricule, caractérisé en ce que :

- un écart dRR sur les intervalles RR est calculé,

- un écart dPR sur les intervalles PR est calculé, l'intervalle PR étant mesuré entre une onde R et l'onde P précédant immédiatement ladite onde R,

- un signal correspondant à une tachycardie ventriculaire est déclenché lorsque l'écart dPR excède l'écart dRR d'une première valeur déterminée, absolue ou relative ;

- un signal correspondant à une tachycardie supraventriculaire est déclenché lorsque l'écart dRR excède l'écart dPR d'une deuxième valeur déterminée, absolue ou relative.

Selon d'autres caractéristiques de l'invention :

- l'écart dPR sur les intervalles PR est mesuré cycle à cycle par la valeur absolue de la différence entre deux intervalles PR successifs : $dPR = ABS|PR_i - PR(i-1)|$ ;

- la valeur retenue pour l'écart dPR sur les intervalles PR est la valeur maximale de l'écart dPR mesuré dans une fenêtre glissante de durée programmable.

- l'écart dPR sur les intervalles PR est mesuré à chaque cycle par la différence entre l'intervalle PR le plus grand et l'intervalle PR le plus petit mesurés durant une fenêtre glissante de durée programmable ;

- l'écart dRR sur les intervalles RR est mesuré à chaque cycle par la différence entre l'intervalle RR le plus grand et l'intervalle RR le plus petit mesurés durant une fenêtre glissante de durée programmable ;

- la fenêtre glissante correspond à une durée de 2 à 32 cycles ventriculaires, de préférence égale à 8 ;

- ladite première valeur déterminée est une valeur absolue de seuil S1, ladite deuxième valeur déterminée est une valeur absolue de seuil S2, et la différence des écarts sur les intervalles PR d'une part et RR d'autre part, est mesurée et comparée auxdites valeurs de seuil S1 et S2 .

- chacune des deux valeurs de seuil (S1, S2) est choisie entre 0 et 150ms ;

- les deux valeurs de seuil (S1, S2) sont choisies égales ;

- les deux valeurs de seuil (S1, S2) sont choisies égales à zéro ;

- la différence des écarts sur les intervalles PR et RR est mesurée sur une fenêtre glissante de durée programmable comprise entre 2 et 32 cycles ventriculaires et de préférence comprise entre 8 et 16 cycles ventriculaires ;

- dans ladite fenêtre glissante, un type de tachycardie, ventriculaire ou supra-ventriculaire, est caractérisé lorsque la valeur de seuil correspondante S1 ou S2 respectivement, est franchie un nombre de fois correspondant à un pourcentage déterminé, compris entre 50% et 100%, et de préférence égal à 75%.

L'invention a également pour objet un système d'analyse de l'activité cardiaque, pour appareil implantable de traitement des tachycardies, tel que stimulateur ou défibrillateur, comportant une sonde auriculaire, et une sonde ventriculaire, du type dans lequel sont analysés les signaux en provenance de l'oreillette, les signaux en provenance du ventricule, caractérisé en ce qu'un dispositif à microprocesseur :

- analyse, cycle à cycle dans une fenêtre glissante de durée programmable, les écarts dRR entre intervalles RR et les écarts dPR entre intervalles PR, un intervalle PR étant mesuré entre une onde R et l'onde P précédant immédiatement ladite onde R,

- déclenche un signal correspondant à une tachycardie ventriculaire lorsque l'écart dPR excède l'écart dRR d'une première valeur déterminée, absolue ou relative, et

- déclenche un signal correspondant à une tachycardie supra-ventriculaire lorsque l'écart dRR excède l'écart dPR d'une deuxième valeur déterminée, absolue ou relative.

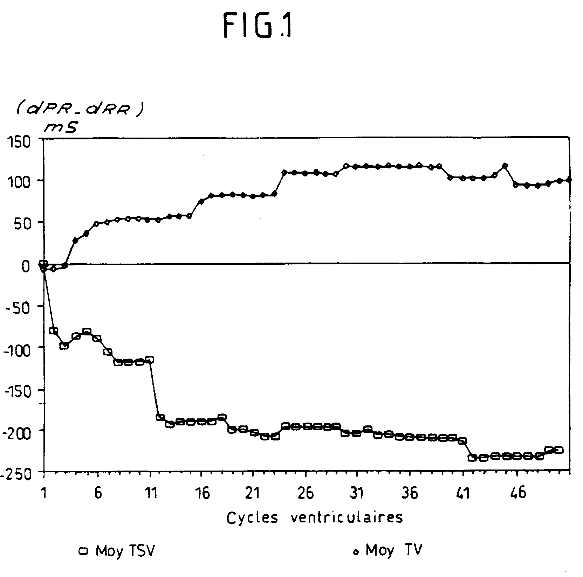

D'autres caractéristiques de l'invention ressortent de la description qui suit faite avec référence à la figure 1.

La figure 1 représente les résultats les mesures (dPR - dRR) en ordonnées, en fonction, en abscisses, du nombre de cycles cardiaques, et ceci sur 20 patients. La différence statistique (dPR - dRR) est négative sur 8 patients présentant les caractéristiques d'une TSV et cette différence statistique est positive sur les 12 patients présentant les caractéristiques d'une TV.

A chaque cycle ventriculaire à l'intérieur d'une fenêtre glissante de 2 à 32 cycles, et de préférence de 8 à 16 cycles, on mesure les intervalles PR et RR. La lettre P est synonyme de l'apparition d'une dépolarisation de l'oreillette (onde P), la lettre R d'une dépolarisation du ventricule (onde R). L'intervalle PR est mesuré entre une onde R et l'onde P qui précède immédiatement ladite onde R.

A l'intérieur de la fenêtre glissante, l'appareil, à l'aide d'un dispositif à microprocesseur par exemple, mesure les écarts dPR des intervalles PR, les écarts dRR des intervalles RR et la différence (dPR-dRR).

les écarts dPR sont mesurés en valeur absolue de la différence cycle à cycle des intervalles PR :

$$dPR = ABS|PR_i - PR(i-1)|$$

On peut prendre comme mesure de l'écart dPR la valeur maximale de l'écart dPR dans une fenêtre glissante de durée programmable.

On peut également prendre comme mesure de l'écart dPR la différence entre l'intervalle PR le plus long et l'intervalle PR le plus court dans la fenêtre glissante. L'écart est alors

$$dPR = (PRmax - PRmin).$$

Les écarts dRR sont mesurés par différence entre l'intervalle RR le plus long et l'intervalle RR le plus court dans la fenêtre glissante, soit

$$dRR = (RRmax - RRmin).$$

La différence (dPR-dRR) est alors comparée à deux valeurs de seuil, S1 et S2, comprises chacune, en valeur absolue, entre O et 150ms.

La valeur de seuil S1 est positive ou nulle.
La valeur de seuil S2 est négative ou nulle.
Les valeurs de seuil S1 et S2 peuvent être différentes en valeur absolue, soit par exemple

$$S1 = +50ms ; \qquad S2 = -30ms.$$

Elles peuvent être égales en valeur absolue, soit par exemple

$$S1 = 30ms ; \qquad S2 = -30ms.$$

Elles peuvent être égales entre elles et égales à zéro, les deux lignes de seuil étant confondues avec l'axe des abscisses comme sur la Fig. 1.

Lorsque la différence (dPR-dRR) est supérieure à la première valeur de seuil S1, une tachycardie ventriculaire est caractérisée pour le cycle courant.

Lorsque la différence (dPR-dRR) est inférieure à la seconde valeur de seuil S2, une tachycardie supra-ventriculaire est caractérisée pour le cycle courant.

Dans la fenêtre glissante, de 8 cycles ventriculaires par exemple, une détermination statistique du type de tachycardie peut être envisagée.

Dans ce cas, si pour la largeur de la fenêtre glissante, par exemple sur 8 cycles ventriculaires, la différence (dPR-dRR) franchit une valeur de seuil par exemple S1 un nombre de fois correspondant à un pourcentage déterminé, par exemple 75%, la tachycardie correspondante (tachycardie ventriculaire dans cet exemple) peut être considérée comme caractérisée.

Le système selon l'invention se compose essentiellement d'un dispositif à deux sondes cardiaques, l'une auriculaire, l'autre ventriculaire, et d'un logiciel pour le calcul automatique de la différence (dPR - dRR).

Dans l'exemple décrit ci-dessus, les valeurs de seuil S1 et S2 correspondent à des valeurs déterminées absolues.

Selon l'invention, ces valeurs peuvent aussi être relatives, et correspondre respectivement à (dPR-dRR)/dRR et (dRR-dPR)/dPR.

## Revendications

1. Procédé d'analyse de l'activité cardiaque, pour dispositif implantable de traitement des tachycardies, du type dans lequel sont analysés les signaux en provenance de l'oreillette, et les signaux en provenance du ventricule, caractérisé en ce que :

   - un écart dRR sur les intervalles RR est calculé,

   - un écart dPR sur les intervalles PR est calculé, l'intervalle PR étant mesuré entre une onde R et l'onde P précédant immédiatement ladite onde R,

   - un signal correspondant à une tachycardie ventriculaire est déclenché lorsque l'écart dPR excède l'écart dRR d'une première valeur déterminée, absolue ou relative ;

   - un signal correspondant à une tachycardie supra-ventriculaire est déclenché lorsque l'écart dRR excède l'écart dPR d'une deuxième valeur déterminée, absolue ou relative.

2. Procédé selon la revendication 1, caractérisé en ce que l'écart dPR sur les intervalles PR est mesuré cycle à cycle par la valeur absolue de la différence entre deux intervalles PR successifs : $dPR = ABS|PRi-PR(i-1)|$.

3. Procédé selon la revendication 2, caractérisé en ce que la valeur retenue pour l'écart dPR sur les intervalles PR est la valeur maximale de l'écart dPR mesuré dans une fenêtre glissante de durée programmable.

4. Procédé selon la revendication 1, caractérisé en ce que l'écart dPR sur les intervalles PR est mesuré à chaque cycle par la différence entre l'intervalle PR le plus grand et l'intervalle PR le plus petit mesurés durant une fenêtre glissante de durée programmable.

5. Procédé selon la revendication 1, caractérisé en ce que l'écart dRR sur les intervalles RR est mesuré à chaque cycle par la différence entre l'intervalle RR le plus grand et l'intervalle RR le plus petit mesurés durant une fenêtre glissante de durée programmable.

6. Procédé selon l'une des revendications 3 à 5, caractérisé en ce que la fenêtre glissante correspond à une durée de 2 à 32 cycles ventriculaires, de préférence égale à 8.

7. Procédé selon la revendication 1, caractérisé en ce que ladite première valeur déterminée est une valeur absolue de seuil S1, ladite deuxième valeur déterminée est une valeur absolue de seuil S2, et la différence des écarts sur les intervalles PR d'une part et RR d'autre part, est mesurée et comparée auxdites valeurs de seuil S1 et S2.

8. Procédé selon la revendication 1, caractérisé en ce que chacune des deux valeurs de seuil (S1, S2) est choisie entre 0 et 150ms.

9. Procédé selon la revendication 8, caractérisé en ce que les deux valeurs de seuil (S1, S2) sont choisies égales.

10. Procédé selon la revendication 9, caractérisé en ce que les deux valeurs de seuil (S1, S2) sont choisies égales a zéro.

11. Procédé selon la revendication 7, caractérisé en ce que la différence des écarts sur les intervalles PR et RR est mesurée sur une fenêtre glissante de durée programmable comprise entre 2 et 32 cycles ventriculaires et de préférence comprise entre 8 et 16 cycles ventriculaires.

12. Procédé selon la revendication 11, caractérisé en ce que dans ladite fenêtre glissante, un type de tachycardie, ventriculaire ou supra ventriculaire, est caractérisé lorsque la valeur de seuil correspondante S1 ou S2 respectivement, est franchie un nombre de fois correspondant à un pourcentage déterminé, de préférence égal à 75%.

13. Système d'analyse de l'activité cardiaque, pour appareil implantable de traitement des tachycardies, tel que stimulateur ou défibrillateur, comportant une sonde auriculaire, et une sonde ventriculaire, du type dans lequel sont analysés les signaux en provenance de l'oreillette, et les signaux en provenance du ventricule, caractérisé en ce qu'un dispositif à microprocesseur :

- analyse cycle à cycle dans une fenêtre glissante de durée programmable les écarts dRR entre intervalles RR et les écarts dPR entre intervalles PR, un intervalle PR étant mesuré entre une onde R et l'onde P précédant immédiatement ladite onde R,

- déclenche un signal correspondant à une tachycardie ventriculaire lorsque l'écart dPR excède l'écart dRR d'une première valeur déterminée, absolue ou relative, et

- déclenche un signal correspondant à une tachycardie supra-ventriculaire lorsque l'écart dRR excède l'écart dPR d'une deuxième valeur déterminée, absolue ou relative.

**Claims**

1. A method for analysis of cardiac activity, for an implantable device for treating tachycardias, of the type in which the signals coming from the atrium and the signals coming from the ventricle are analysed, characterised in that:

   - a dRR deviation on the R-R intervals is calculated,
   - a dPR deviation on the P-R intervals is calculated, the P-R interval being measured between a wave R and the wave P immediately preceding said wave R,
   - a signal corresponding to a ventricular tachycardia is triggered when the dPR deviation exceeds the dRR deviation by a first absolute or relative given value,
   - a signal corresponding to a supra-ventricular tachycardia is triggered when the dRR deviation exceeds the dPR deviation by a second absolute or relative given value.

2. A method according to Claim 1, characterised in that the dPR deviation over the P-R intervals is measured cycle by cycle by the absolute value of the difference between two successive P-R intervals:

   $$dPR = ABS|PRi-PR(i-1)|.$$

3. A method according to Claim 2, characterised in that the value retained for the dPR deviation over the P-R intervals is the maximum value of the dPR deviation measured in a sliding window of programmable duration.

4. A method according to Claim 1, characterised in that the dPR deviation over the P-R intervals is measured on each cycle by the difference between the largest P-R interval and the smallest P-R interval, measured during a sliding window of programmable duration.

5. A method according to Claim 1, characterised in that the dRR deviation over the R-R intervals is measured on each cycle by the difference between the largest R-R interval and the smallest R-R interval, measured during a sliding window of programmable duration.

6. A method according to one of Claims 3 to 5, characterised in that the sliding window corresponds to a duration of 2 to 32 ventricular cycles, preferably equal to 8.

7. A method according to Claim 1, characterised in that said first given value is an absolute threshold value S1, said second given value is an absolute threshold value S2, and the difference between the deviations over the P-R interval on one hand and the R-R interval on the other hand is measured and compared with said threshold values S1 and S2.

8. A method according to Claim 1, characterised in that each of the two threshold values (S1, S2) is selected between 0 and 150 ms.

9. A method according to Claim 8, characterised in that the two threshold values (S1, S2) are selected to be equal.

10. A method according to Claim 9, characterised in that the two threshold values (S1, S2) are selected to be equal to zero.

11. A method according to Claim 7, characterised in that the difference in the deviations over the P-R and R-R intervals is measured over a sliding window of programmable duration of between 2 and 32 ventricular cycles and preferably of between 8 and 16 ventricular cycles.

12. A method according to Claim 11, characterised in that in said sliding window a type of tachycardia, ventricular or supra-ventricular, is characterised when the corresponding threshold value S1 or S2 respectively is exceeded a number of times corresponding to a given percentage, preferably equal to 75%.

13. A system for analyzing cardiac activity, for an implantable apparatus for treating tachycardias, such as a pacemaker or defibrillator, comprising an atrial sensor and a ventricular sensor, of the type in which the signals coming from the atrium and the signals coming from the ventricle are analyzed, characterised in that a microprocessor device:

- analyses cycle by cycle in a sliding window of programmable duration the dRR deviations between R-R intervals and the dPR deviations between P-R intervals, a P-R interval being measured between a wave R and the wave P immediately preceding said wave R,
- triggers a signal corresponding to a ventricular tachycardia when the dPR deviation exceeds the dRR deviation by a first absolute or relative given value, and
- triggers a signal corresponding to a supra-ventricular tachycardia when the dRR deviation exceeds the dPR deviation by a second absolute or relative given value.

## Patentansprüche

1. Verfahren zur Analyse der Herztätigkeit für eine implantierbare Vorrichtung zur Behandlung von Tachykardien, von dem Typ, in dem die Signale, die von dem Vorhof herkommen, und die Signale, die von der Herzkammer herkommen, analysiert werden, dadurch gekennzeichnet, daß:

- eine Abweichung dRR auf den Intervallen RR berechnet wird,

- eine Abweichung dPR auf den Intervallen PR berechnet wird, wobei das Intervall PR zwischen einer R-Welle und der P-Welle, die unmittelbar der R-Welle vorausgeht, gemessen wird,

- ein Signal ausgelöst wird, das einer ventrikulären Tachykardie entspricht, wenn die Abweichung dPR die Abweichung dRR um einen ersten bestimmten Wert übersteigt, absolut oder relativ;

- ein Signal ausgelöst wird, das einer supraventrikulären Tachykardie entspricht, wenn die Abweichung dRR die Abweichung dPR um einen zweiten bestimmten Wert übersteigt, absolut oder relativ.

2. Verfahren nach Anspruch 1, dadurch gekennzeichnet, daß die Abweichung dPR auf den Intervallen PR Zyklus für Zyklus durch den Absolutwert der Differenz zwischen zwei aufeinanderfolgenden PR-Intervallen gemessen wird:

$$dPR = ABS|PR_i - PR_{(i-1)}|.$$

3. Verfahren nach Anspruch 2, dadurch gekennzeichnet, daß der für die Abweichung dPR auf den Intervallen PR erhaltene Wert der Maximalwert der Abweichung dPR ist, der in einem verschiebbaren Fenster von programmierbarer Dauer gemessen worden ist.

4. Verfahren nach Anspruch 1, dadurch gekennzeichnet, daß die Abweichung dPR auf den Intervallen PR in jedem Zyklus durch die Differenz zwischen dem größten Intervall PR und dem kleinsten Intervall PR, die während einem verschiebbaren Fenster von programmierbarer Dauer gemessen wurden, gemessen wird.

5. Verfahren nach Anspruch 1, dadurch gekennzeichnet, daß die Abweichung dRR auf den Intervallen RR in jedem Zyklus durch die Differenz zwischen dem größten Intervall RR und dem kleinsten Intervall RR, die während einem verschiebbaren Fenster von programmierbarer Dauer gemessen wurden, gemessen wird.

6. Verfahren nach einem der Ansprüche 3 bis 5, dadurch gekennzeichnet, daß das verschiebbare Fenster einer Dauer von 2 bis 32 ventrikulären Zyklen, vorzugsweise 8 entspricht.

7. Verfahren nach Anspruch 1, dadurch gekennzeichnet, daß der erste bestimmte Wert ein absoluter Schwellenwert S1 ist, der zweite bestimmte Wert ein absoluter Schwellenwert S2 ist, und die Differenz der Abweichungen auf den Intervallen PR einerseits und RR andererseits gemessen und jeweils mit den Schwellenwerten S1 und S2 verglichen wird.

8. Verfahren nach Anspruch 1, dadurch gekennzeichnet, daß jeder der zwei Schwellenwerte (S1, S2) zwischen 0 und 150 ms ausgewählt wird.

9. Verfahren nach Anspruch 8, dadurch gekennzeichnet, daß die zwei Schwellenwerte (S1, S2) als gleich ausgewählt werden.

10. Verfahren nach Anspruch 9, dadurch gekennzeichnet, daß die zwei Schwellenwerte (S1, S2) als gleich Null ausgewählt werden.

11. Verfahren nach Anspruch 7, dadurch gekennzeichnet, daß die Differenz der Abweichungen auf den Intervallen PR und RR auf einem verschiebbaren Fenster mit programmierbarer Dauer mit zwischen einschließlich 2 und 32 ventrikulären Zyklen und vorzugsweise mit zwischen einschließlich 8 und 16 ventrikulären Zyklen gemessen wird.

12. Verfahren nach Anspruch 11, dadurch gekennzeichnet, daß in dem verschiebbaren Fenster ein Tachykardientyp, ventrikulär oder supraventrikulär, charakterisiert wird, wenn der Schwellenwert entsprechend jeweils S1 oder S2 eine Anzahl an Malen übersteigt, die einen bestimmten Prozentsatz entspricht, vorzugsweise 75%.

13. System zur Analyse der Herztätigkeit für ein implantierbares Gerät zur Behandlung von Tachykardien, wie eine Anregungsvorrichtung oder einen Defibrillator, umfassend einen Vorhofkatheter und einen Herzkammerkatheter, von dem Typ, in dem die Signale, die von dem Vorhof kommen und die Signale, die von der Herzkammer kommen, analysiert werden, dadurch gekennzeichnet, daß eine Mikroprozessorvorrichtung:

-   Zyklus für Zyklus in einem verschiebbaren Fenster mit programmierbarer Dauer die Abweichungen dRR zwischen Intervallen RR und Abweichungen dPR zwischen Intervallen PR analysiert, wobei ein Intervall PR zwischen einer R-Welle und der P-Welle, die unmittelbar der R-Welle vorausgeht, gemessen wird,

-   ein Signal auslöst, das einer ventrikulären Tachykardie entspricht, wenn die Abweichung dPR die Abweichung dRR um einen ersten bestimmten Wert übersteigt, absolut oder relativ, und

-   ein Signal auslöst, das einer supraventrikulären Tachykardie entspricht, wenn die Abweichung dRR die Abweichung dPR um einen zweiten bestimmten Wert übersteigt, absolut oder relativ.

# FIG.1